(19) Europäisches Patentamt
European Patent Office
Office européen des brevets

(11) Veröffentlichungsnummer: **0 009 178**
**B1**

(12) # EUROPÄISCHE PATENTSCHRIFT

(45) Veröffentlichungstag der Patentschrift: **23.06.82**

(51) Int. Cl.³: **G 03 C 7/36**, C 07 B 29/00

(21) Anmeldenummer: **79103309.5**

(22) Anmeldetag: **06.09.79**

(54) **Verfahren zur Herstellung von 2-Äquivalent-Gelbkupplern.**

(30) Priorität: **16.09.78 DE 2840381**

(43) Veröffentlichungstag der Anmeldung: **02.04.80 Patentblatt 80/7**

(45) Bekanntmachung des Hinweises auf die Patenterteilung: **23.06.82 Patentblatt 82/25**

(84) Benannte Vertragsstaaten: **BE DE FR GB**

(56) Entgegenhaltungen:
**DE-A-2 528 860**
**FR-A-2 296 805**
**FR-A-2 327 574**
**GB-A-1 420 564**
**M. FIESER et al.: »Reagents for Organic Synthesis«, Band 2, 1969, Seite 473, Ausg. John Wiley, New York, USA**
**M. FIESER et al.: »Reagents for Organic Synthesis«, Band 4, 1974, Ausg. John Wiley, New York, USA**

(73) Patentinhaber: **AGFA-GEVAERT Aktiengesellschaft, D-5090 Leverkusen 1 (DE)**

(72) Erfinder: **Renner, Günter, Dr., Roggendorfstrasse 65, D-5000 Koeln 80 (DE)**
Erfinder: **Scheben, Quirin, Schnellweiderstrasse 76, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 009 178 B1

BUNDESDRUCKEREI BERLIN

# Verfahren zur Herstellung von 2-Äquivalent-Gelbkupplern

Die Erfindung betrifft ein neues Verfahren zur Herstellung von 2-Äquivalent-Gelbkupplern.

In der subtraktiven Drei-Farben-Silberhalogenidfotografie ist es gängige Praxis, Farbkuppler zu verwenden, die bei der Farbentwicklung des belichteten fotografischen Materials mit der oxidierten Entwicklersubstanz, insbesondere einer aromatischen, primäre Aminogruppen enthaltenden Verbindung, kuppeln unter Bildung eines Blaugrün-, Purpur- und Gelfarbstoffbildes. Für die Erzeugung des Gelbfarbstoffbildes werden offenkettige Ketomethylenverbindungen (vgl. Mees & James, »The Theory of the Photographic Process«, 3. Auflage, 1966, Seiten 388 – 389), vorzugsweise Acylacetamide, z. B. Acylacetanilide, verwendet.

Es ist auch bekannt, Gelbkuppler zu verwenden, in denen die Methylengruppe unsubstituiert ist, bei denen für die Erzeugung eines Moleküls Farbstoff daher die Entwicklung von 4 Äquivalenten Silberhalogenid erforderlich ist, sowie Gelbkuppler, bei denen die Methylengruppe einen Substituenten trägt, der bei der Farbentwicklung abgespalten wird, so daß zur Bildung von 1 Molekül Farbstoff nur 2 Äquivalente Silberhalogenid entwickelt werden müssen. Erstere Verbindungen sind unter der Bezeichnung »4-Äquivalent-Kuppler« bekannt, letztere unter der Bezeichnung »2-Äquivalent-Kuppler«.

Die Hauptvorteile von 2-Äquivalent-Farbkupplern sind bekannt. Sie benötigen nur etwa halb soviel Silberhalogenid wie die 4-Äquivalent-Kuppler, so daß bei der Herstellung der fotografischen Materialien weniger Silberhalogenid benötigt wird und dünnere Silberhalogenidemulsionsschichten verwendet werden können, was zu einer verbesserten Auflösung und Schärfe führt.

So sind beispielsweise in der FR-A-1 411 384 2-Äquivalent-Gelbkuppler beschrieben, deren aktive Methylengruppe eine Aroxygruppe, insbesondere einen Phenoxysubstituenten, trägt. Diese 2-Äquivalent-Kuppler werden hergestellt durch Umsetzung des entsprechenden Kupplers, der in kuppelnder Stellung (in der aktiven Methylengruppe) ein Chloroatom substituiert enthält, mit Phenolen in Gegenwart von Triäthylamin als basischem Kondensationsmittel in Acetonitril als Lösungsmittel. Dieses Verfahren führt jedoch zur Bildung von vielen Nebenprodukten, so daß die Isolierung des phenoxysubstituierten Kupplers schwierig ist und geringe Ausbeuten erhalten werden.

Andere bekannte 2-Äquivalent-Gelbkuppler, die in der kuppelnden Stellung über Stickstoff verknüpft einen Heterocyclus tragen (vgl. z. B. DE-A-2 318 807, 2 329 587 und 2 363 675) werden hergestellt durch Umsetzung des entsprechenden an der Methylengruppe chlor- oder bromsubstituierten Kupplers mit dem geeigneten Heterocyclus in einem aprotischen Lösungsmittel, z. B. in Dimethylformamid, Hexamethylphosphorsäuretriamid oder Dimethylsulfoxid, in Gegenwart einer Base, wobei als Base aliphatische Amine, wie Triäthylamin oder Tetramethylguanidin, basische heterocyclische Verbindungen, wie Pyridin, oder Alkalisalze von Alkoholen, wie Natriummethylat, verwendet werden. Diese Verfahren sind jedoch ebenfalls von unerwünschten Nebenreaktionen begleitet.

Der Erfindung liegt die Aufgabe zugrunde, 2-Äquivalent-Gelbkupplerverbindungen in besseren Ausbeuten und unter Bildung von einer möglichst geringen Anzahl von Nebenprodukten herzustellen.

Es wurde nun gefunden, daß 2-Äquivalent-Acylacetamid-Gelbkuppler, d. h. in $\alpha$-Stellung mit einer organischen Abgangsgruppe substituierte Acylacetamide durch Umsetzung von $\alpha$-Halogenacylacetamidkupplern, die also in der kuppelnden Stellung (in der Methylengruppe) ein Halogenatom, insbesondere ein Chloratom oder ein Bromatom aufweisen, mit einer die Abgangsgruppe liefernden organischen Verbindung vorzugsweise in einem aprotischen Lösungsmittel unter Verwendung von

1,5-Diazabicyclo[4,3,0]non-5-en (DBN) oder
1,8-Diazabicyclo[5,4,0]undec-7-en (DBU)

als basische Kondensationsmittel in hohen Ausbeuten und in hoher Reinheit hergestellt werden können.

Durch Verwendung der genannten basischen Kondensationsmittel ist es möglich, in die kuppelnde Stellung der Gelbkuppler die verschiedensten organischen Substituenten einzuführen, die von organischen Verbindungen mit variierender Säurestärke einschließlich Phenolen (wie sie beispielsweise in der obengenannten französischen Patentschrift beschrieben sind) und NH-aciden organischen Verbindungen, insbesondere heterocyclischen Verbindungen mit mindestens einer sauren NH-Gruppierung im Ring (wie sie beispielsweise in den obengenannten deutschen Offenlegungsschriften beschrieben sind) abgeleitet sind, von denen in der nachfolgenden Tabelle I einige repräsentative Beispiele angegeben sind. Zum Begriff »NH-acide organische Verbindung« wird verwiesen auf H. A. Staab, »Einführung in die Theoretische Organische Chemie«, Verlag Chemie, Weinheim, 1959, Seite 627 ff.

Tabelle I

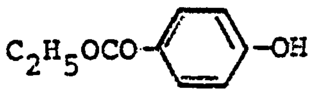

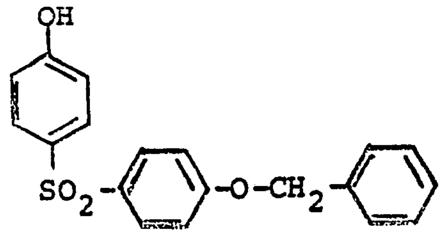

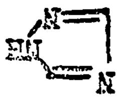

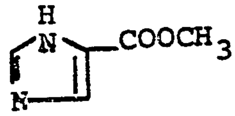

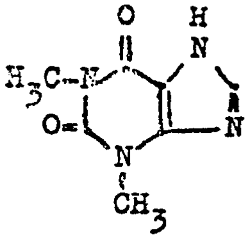

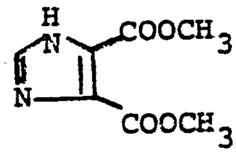

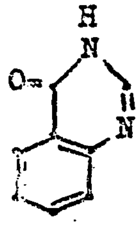

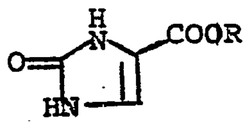

$R = CH_3, C_2H_5$
$i-C_4H_9$

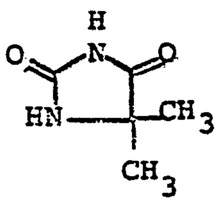

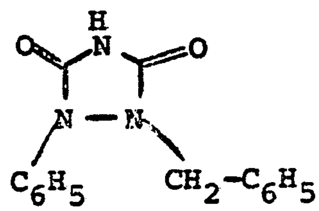

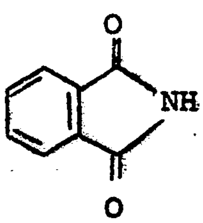

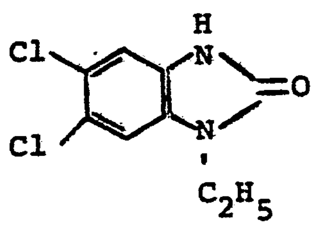

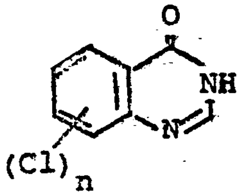

n = 1-3

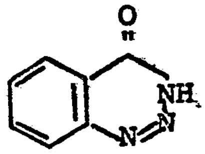

Fortsetzung

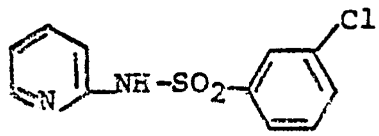

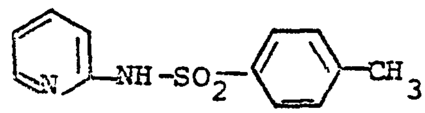

Nach dem erfindungsgemäßen Verfahren läuft die Substitution sehr selektiv ab, so daß wenig Nebenprodukte gebildet und höhere Ausbeuten als bei den bekannten Verfahren erhalten werden. Die isolierten Reaktionsprodukte weisen einen hohen Reinheitsgrad auf.

Verschiedene basische Kondensationsmittel für die Herstellung von 2-Äquivalentgelbkupplern sind in der DE-A-2 528 860 beschrieben, darunter auch Diazabicyclooctan (DABCO). Es findet sich dort jedoch kein Hinweis auf die erfindungsgemäß zu verwendenden basischen Kondensationsmittel DBN und DBU sowie deren überlegene Wirkung bei dem erfindungsgemäßen Herstellungsverfahren. Eine dieser Substanzen (DBN) ist zwar in Fieser-Fieser, »Reagents for Organic Synthesis«, Bd. 2, S. 473, Zeile 27, neben einer Reihe von anderen Verbindungen als »condensation catalyst« genannt; doch geht hieraus nicht die Art der in Betracht gezogenen Kondensationsreaktion hervor. Unter anderem sind an dieser Stelle auch saure Reagentien genannt. In einer weiteren Textstelle des genannten Werkes (Bd. 4, S. 16 und 17) sind beide erfindungsgemäß verwendete Verbindungen erwähnt, hier jedoch im Zusammenhang mit der Dehydrohalogenierungsreaktion. Da es sich hierbei um einen Reaktionstyp handelt, der völlig verschieden ist von demjenigen, der dem erfindungsgemäßen Verfahren zugrunde liegt, konnten von einer Eignung bestimmter Substanzen als Dehydrohalogenierungsmittel keine Rückschlüsse auf deren Brauchbarkeit bei dem erfindungsgemäßen Verfahren gezogen werden.

Erfindungsgemäß werden insbesondere $\alpha$-Halogen-$\alpha$-acylacetamide, der allgemeinen Formel

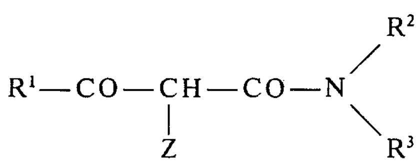

umgesetzt, worin bedeuten:

Z ein Halogenatom, insbesondere ein Chloratom oder ein Bromatom,

$R^1$ eine tert. Butylgruppe oder eine Arylgruppe, vorzugsweise eine Phenylgruppe, die einen oder mehrere Substituenten, wie z. B. $C_1-C_{18}$-Alkyl, $C_1-C_{18}$-Alkoxy, Aralkyl, Aryl, Aroxy, Sulfo, Carboxy, Halogen, wie Chlor, Brom und Fluor, Acyl, Acyloxy, Acylamino oder Amino tragen kann,

$R^2$ Wasserstoff oder eine $C_1-C_5$-Alkylgruppe, wie Methyl und

$R^3$ Aryl, wie Phenyl, das durch einen oder mehrere Substituenten, wie z. B. $C_1-C_{18}$-Alkyl, $C_1-C_{18}$-Alkoxy, Halogen, wie Chlor, Brom und Fluor, Sulfo, Carboxy, Aryl, Aralkyl, Aroxy, Acyl, Acyloxy, Acylamino oder Amino substituiert sein kann.

Soweit oben bei der Definition von $R^1$ und $R^3$ auf Acylreste Bezug genommen wird, leiten sich diese ab von organischen Säuren wie aliphatischen oder aromatischen Carbon- oder Sulfonsäuren unter Einbeziehung von Kohlensäurehalbestern, Carbaminsäuren und Sulfaminsäuren, wobei die beiden letzteren am Stickstoffatom durch Alkyl, Aryl, Aralkyl oder einen Heterocyclus substituiert sein können.

Die nach dem erfindungsgemäßen Verfahren hergestellten 2-Äquivalent-Farbkuppler sind natürlich vorzugsweise von bekannten 4-Äquivalent-Farbkupplern mit ausgezeichneten Eigenschaften in bezug auf die Absorptionseigenschaften und die Stabilität der bei der Farbentwicklung gebildeten Farbstoffe abgeleitet.

Bevorzugt sind Pivaloylacetanilide und Benzoylacetanilide, insbesondere o-Alkoxy-benzoylacetanilide, die in dem Anilinrest 1 bis 3 Substituenten des vorstehend angegebenen Typs, vorzugsweise in den 2-, 4- und 5-Stellungen, tragen können.

In dem erfindungsgemäßen Verfahren werden die Kuppler mit einer chlor- oder bromsubstituierten

Methylengruppe und die die Abgangsgruppe liefernden Verbindungen im allgemeinen in äquimolaren Mengen verwendet, obgleich die zuletzt genannten Verbindungen auch im Überschuß verwendet werden können.

Die als basisches Kondensationsmittel verwendete stickstoffhaltige Base kann in bezug auf die die Abgangsgruppe liefernde Verbindung in äquimolaren Mengen verwendet werden. Vorzugsweise wird jedoch ein Molverhältnis von stickstoffhaltiger Base zu der die Abgangsgruppe liefernden Verbindung von 2 oder mehr angewendet.

Die Substitutionsreaktion läuft im allgemeinen ab beim Erwärmen der Reaktionspartner in einem geeigneten Lösungsmittel, wie Acetonitril, Butanon-2, Aceton, Dimethylformamid, Dimethylacetamid, N-Methyl-pyrrolidon, Hexamethylphosphorsäuretriamid (HEMPA) u. a. auf 40—100°C (vorzugsweise 50—80°C). Die Lösungsmittelmenge ist nicht kritisch und hängt von der Löslichkeit der Reaktionspartner darin ab.

Die Umsetzung wird zweckmäßigerweise so ausgeführt, daß man den nukleophilen Reaktionspartner, d. i. die die Abgangsgruppe liefernde Verbindung zusammen mit DBN oder DBU, gelöst in einem aprotischen, vorzugsweise dipolaren Lösungsmittel, bei der Reaktionstemperatur vorlegt und das $\alpha$-halogensubstituierte $\beta$-Ketoacylanilid gegebenenfalls in gleicher Weise gelöst zugibt.

Es ist jedoch auch möglich, die genannten stickstoffhaltigen Basen langsam zu einer Lösung der die Abgangsgruppe liefernden Verbindung und des in $\alpha$-Stellung halogenierten Gelbkupplers zuzutropfen. Ebenso kann nach der in DE-A-2 545 756 beschriebenen Methode mittels der erfindungsgemäß verwendeten stickstoffhaltigen Basen eine Umsetzung von in der Kupplungsstelle nicht substituierten 4-Äquivalent-Kupplern zu den gewünschten 2-Äquivalent-Kupplern herbeigeführt werden, wobei vermutlich in $\alpha$-Stellung halogenierte Kuppler nur als Intermediärprodukt auftritt, das aber nicht isoliert wird.

Die Reaktionsbedingungen (Reaktionstemperatur) werden vorzugsweise so gewählt, daß die Reaktionsgeschwindigkeit der Umsetzung des nukleophilen Reaktionspartners mit dem in $\alpha$-Stellung halogenierten Kupplers möglichst groß ist. Da eine längere Verweilzeit des halogenierten Kupplers im alkalischen Reaktionsmedium häufig von Nachteil ist, ist Wert darauf zu legen, daß die beiden Reaktionspartner rasch miteinander reagieren.

Der nach dem erfindungsgemäßen Verfahren hergestellte 2-Äquivalent-Gelbkuppler kann auf übliche Weise aus dem Reaktionsmedium isoliert werden, indem man die Reaktionsmischung in Wasser, Essigsäure, verdünnte Schwefelsäure oder verdünnte Salzsäure gießt und umkristallisiert, wobei man einen Farbkuppler mit einem hohen Reinheitsgrad erhält. Bei einigen Verbindungen wird der Umkristallisation eine Extraktionsstufe vorgeschaltet.

Repräsentative Beispiele für nach dem erfindungsgemäßen Verfahren herstellbare 2-Äquivalent-Farbkuppler sind in der folgenden Tabelle II angegeben. Das Herstellungsverfahren wird durch die weiter unten angegebenen Herstellungsbeispiele erläutert.

<u>Tabelle II</u>

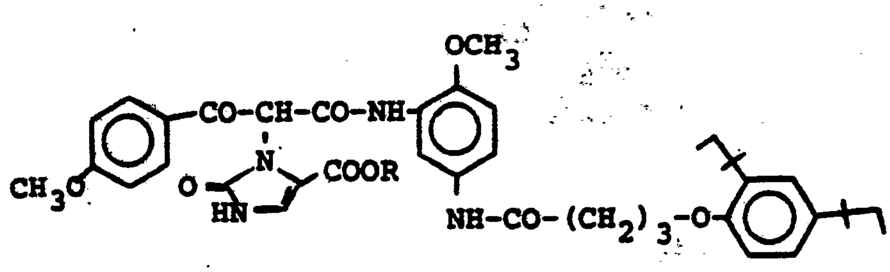

| | | | |
|---|---|---|---|
| 1. | R = $CH_3$ | F = 185-87°C | Ausb. 76 % |
| 2. | $C_2H_5$ | = 154-57°C | Ausb. 73 % |
| 3. | $C_4H_9$ | " 121-25°C | Ausb. 72 % |

Fortsetzung

4.

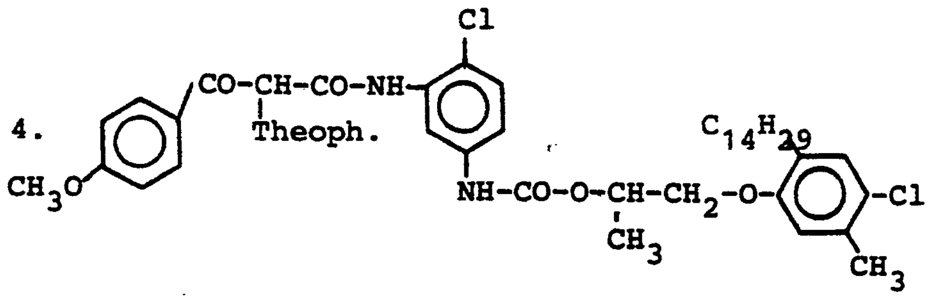

F = 83°C     Ausb. 67 %

5.

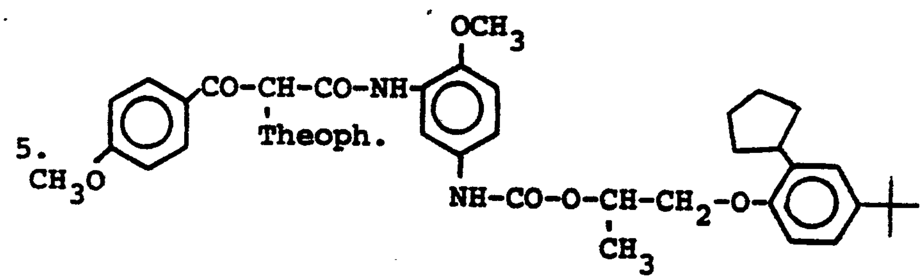

F = 165-67°C     Ausb. 77 %

6.

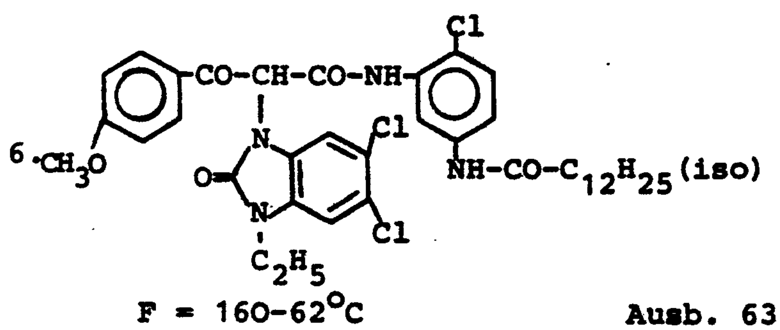

F = 160-62°C     Ausb. 63 %

7.

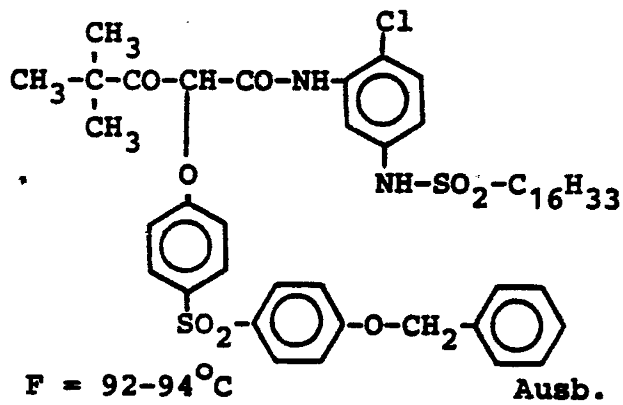

F = 92-94°C     Ausb. 69 %

Fortsetzung

8. 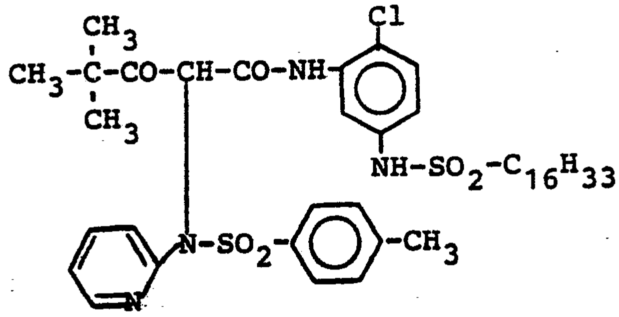

F = 123-125°C Ausb. 61 %

9. 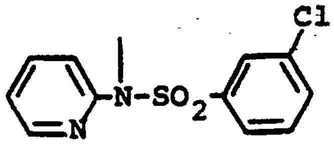

F = 110-12°C Ausb. 63 %

10. 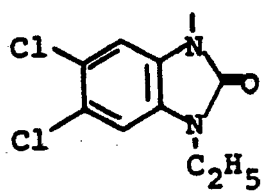

F = 90 - 92°C Ausb. 70 %

11. 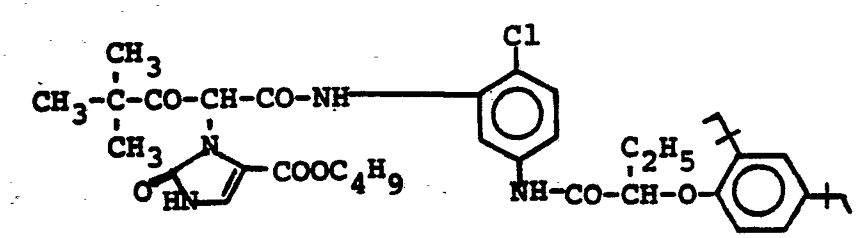

F = 163-65°C Ausb. 53 %

Fortsetzung

12. 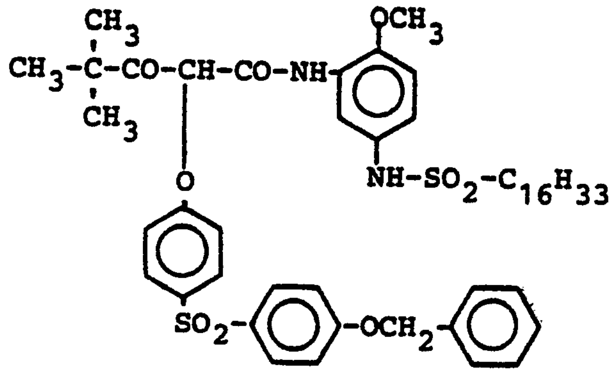

F = 129-30°C Ausb. 64 %

13. 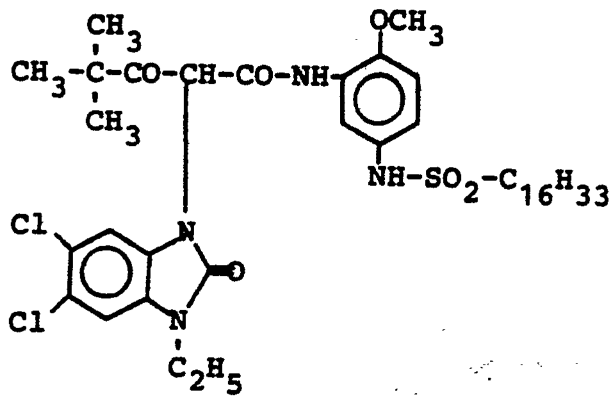

F = 156-58°C Ausb. 81 %

14. 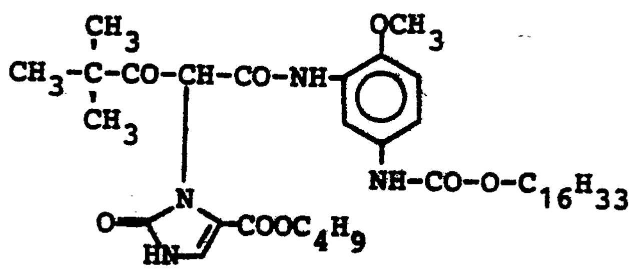

F = 127-129°C Ausb. 84 %

15. 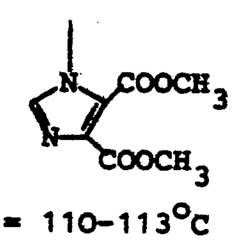

F = 110-113°C Ausb. 77 %

Fortsetzung

16.

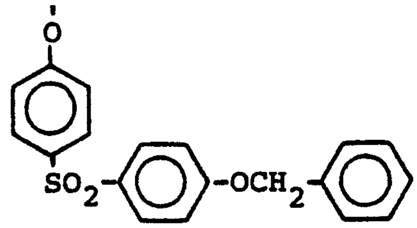

F = 134-36°C Ausb. 75 %

17.

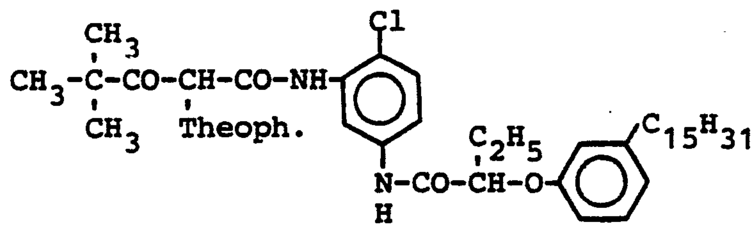

Öl Ausb. 75 %

18.

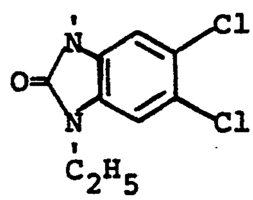

F = 70-72°C Ausb. 53 %

19.

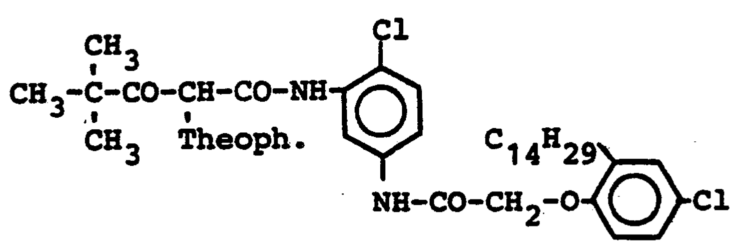

Öl Ausb. 82 %

Fortsetzung

20. 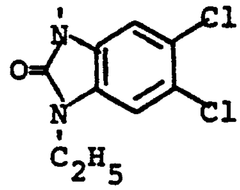

F = 128-29°C  Ausb. 77 %

21. 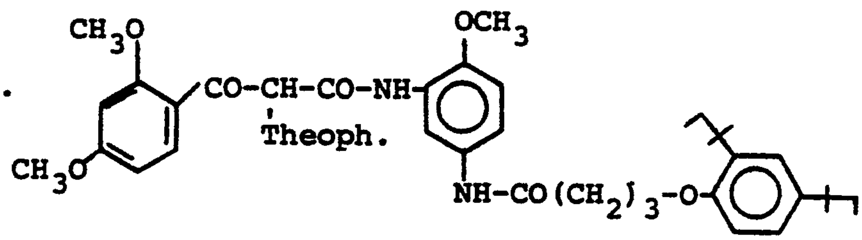

F = 108-111°C  Ausb. 62 %

22. 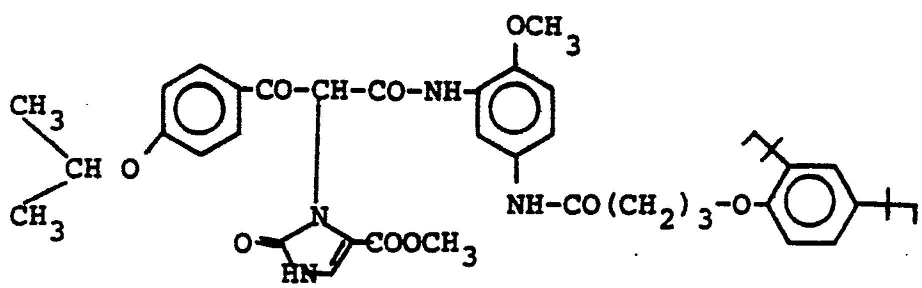

F = 233-36°C  Ausb. 73 %

23. 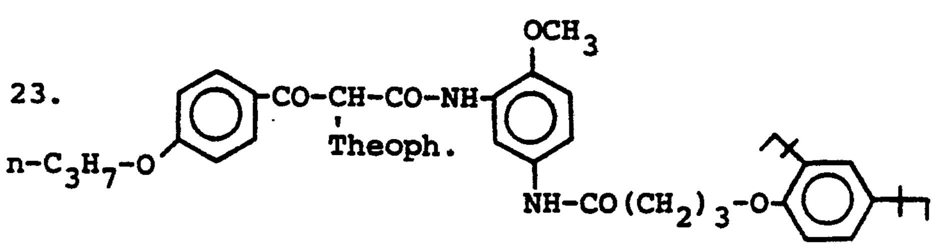

F = 148-50°C  Ausb. 63 %

Fortsetzung

24. 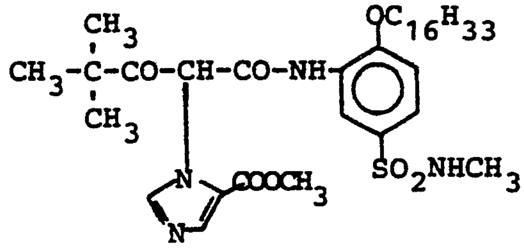

F = 69-72°C Ausb. 86 %

25. 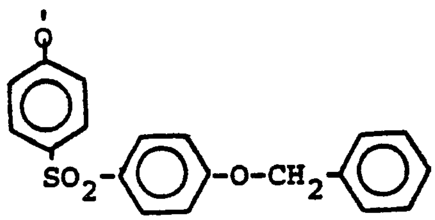

F = 132-35°C Ausb. 72 %

26. 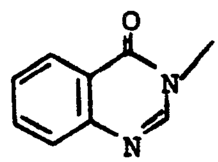

F = 72-74°C Ausb. 88 %

27. 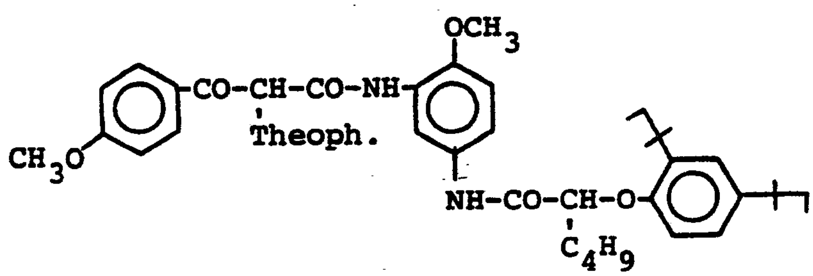

F = 178-81°C Ausb. 86 %

Fortsetzung

28.

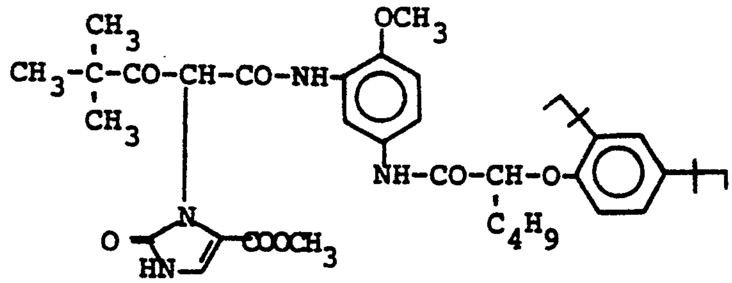

F = 200 - 203 °C

Ausb. 71 %

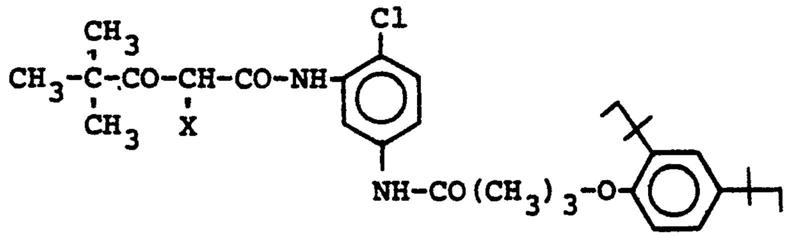

29. X =

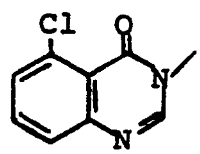

Ausb. 80 %
F = 180-82 °C

30.

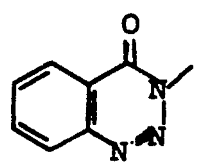

Ausb. 76 %
F = 182-85 °C

31.

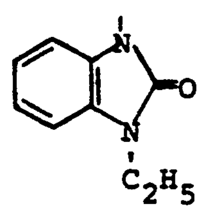

Ausb. 67 %
F = 187-90 °C

32.

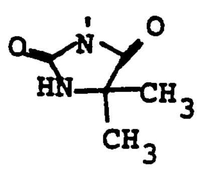

Ausb. 54 %
F = 108-110 °C

Fortsetzung

33. 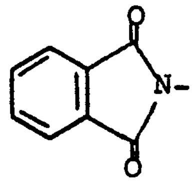

Ausb. 61 %
F = 187-189°C

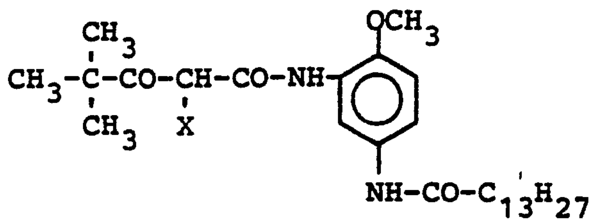

34. X = 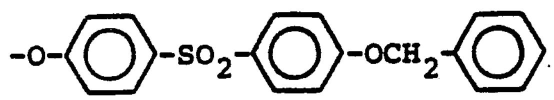

F = 110-12°C Ausb. 68 %

35. X = Theoph.

F = 170-73°C Ausb. 71 %

36. X = 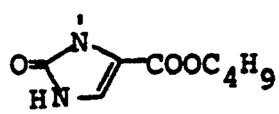

F = 174-75°C Ausb. 62 %

37. X = 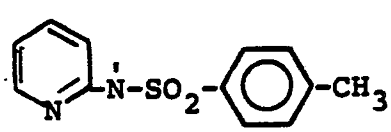

F = 166-68°C Ausb. 52 %

Fortsetzung

Theoph. = Theophyllin

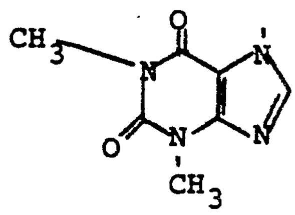

Synthesebeispiele

Kuppler 13

α-(5,5-Dichlor-3-ethyl-benzimidazol-2-on-1-yl)-2-methoxy-5-cetylsulfonamido-pivaloylacetanilid

| | |
|---|---|
| 41 g | α-Pivaloyl-α-chlor-2-methoxy-5-cetylsulfonamidoacetanilid und |
| 21 g | 5,6-Dichlor-3-ethylbenzimidazolon werden in |
| 300 ml | Dimethylacetamid gelöst. Bei 30–40° C tropft man |
| 30 ml | Diazabicycloundecen (DBU) zu. Es wird in Eis/Salzsäure gefällt und aus Alkohol umkristallisiert. |

Man erhält 45,2 g des gewünschten 2-Äquivalent-Kupplers 13.

Kuppler 11

2-Chlor-5-(α-2,4-ditert.-amylphenoxybutyramido)-α-(5-butoxycarbonylimidazolin-2-on-1-yl)-α-pivaloylacetanilid

Man verfährt wie bei der Synthese von Kuppler 13 beschrieben unter Verwendung von

| | |
|---|---|
| 30 g | 2,α-Dichlor-5-(α-2,4-ditert.-amyl-phenoxybutyramido)-α-pivaloyl-acetanilid |
| 10,5 g | 4-Butoxycarbonylimidazolin-2-on und |
| 17 g | Diazabicyclononen (DBN) |

Man erhält Kuppler 11 in 53%iger Ausbeute.

Kuppler 27

2-Methoxy-5-(α-2,4-ditert.-pentylphenoxy-caprylamido)-α-(4,6-dimethyl-5,7-dioxo-4,5,6,7-tetrahydro-1-imidazolo[4,5-d]pyrimidinyl)-α-(4-methoxybenzoyl)-acetanilid

| | |
|---|---|
| 10 g | Theophyllin werden in 100 ml HEMPA mit |
| 19 g | DBU gelöst. Bei 60° C gibt man dazu eine Lösung von 34 g α-(4-Methoxybenzoyl)-α-chlor-2-methoxy-5-(α-2,4-ditert.-pentyl-phenoxy-caprylamido)-acetanilin in 50 ml HEMPA. Man fällt sauer und kristallisiert aus Alkohol um. |

Man erhält den gewünschten Kuppler 27 in 86%iger Ausbeute.

Vergleichsversuche

1. Unter Verwendung der in der nachstehenden Tabelle aufgeführten Basen wurde der Kuppler 4 aus dem entsprechenden in α-Stellung chlorierten Kuppler 2,α-Dichlor-5-[2-(4-chlor-5-methyl-2-tetradecylphenoxy)-isopropoxycarbonyl-amino]-α-(4-methoxybenzoyl)-acetanilid und Theophyllin hergestellt nach folgender Methode.

Zu 0,12 Mol Theophyllin gibt man 100 ml Dimethylacetamid und 0,2 Mol Base. Man erwärmt auf 70° C und gibt 0,1 Mol des chlorierten Kupplers zu. Man rührt in Wasser/Eis/Salzsäure aus, saugt ab und wäscht mit Wasser. Das trockene Rohprodukt wird aus Acetonitril ungelöst.

Die erzielte Ausbeute ist in Tabelle III angegeben.

Tabelle III

| Base | Ausbeute [g] | [%] |
|---|---|---|
| NaOH | 37 | 40,9 |
| $NaOCH_3$ | 36 | 39,8 |
| Tetramethylguanidin (TMG) | 44 | 48,6 |
| LiOH | 39 | 43,1 |
| DBU | 62 | 68,5 |
| tert.-$C_4H_9$-OK | 39 | 43,1 |

2. Bei Wiederholung des Versuches, jedoch mit Acetonitril als Lösungsmittel wurden folgende Ergebnisse erzielt.

| Base | Ausbeute [g] | [%] |
|---|---|---|
| $NaOCH_3$ | 32 | 35,4 |
| TMG | 37 | 40,9 |
| DBU | 43 | 47,5 |

**3. Vergleichbare Ergebnisse wie unter 1 wurden in HEMPA als Lösungsmittel gefunden.**

**4. Zur Herstellung des Kupplers 24 2-Hexadecoxy-α-(5-methoxycarbonyl-imidazolyl)-5-(N-methylsulfamoyl)-α-pivaloylacetanilid verfuhr man wie bei 1 beschrieben, wobei jedoch die die Abgangsgruppe liefernde Verbindung (4-methoxycarbonyl-imidazol) nur in 10%igem Überschuß verwendet wurde und der in α-Stellung chlorierte 2-Äquivalentkuppler bei 50° C zugefügt wurde.**

| Base | Lösungsmittel | Ausbeute [%] |
|---|---|---|
| **TMG** | Dimethylacetamid | 69 |
| **$NaOCH_3$** | Acetonitril | 64 |
| **DBU** | Dimethylacetamid | 86 |

## Patentansprüche

**1. Verfahren zur Herstellung eines in α-Stellung substituierten α-Pivaloylacetanilids oder α-Benzoylacetanilids durch Umsetzung der entsprechenden α-Halogen-Verbindung der Formel**

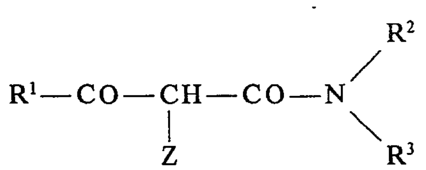

worin bedeuten

Z Halogen,
$R^1$ tert. Butyl oder Aryl,
$R^2$ Wasserstoff oder $C_1-C_5$-Alkyl,
$R^3$ Aryl

mit einem Phenol oder einer NH-aciden organischen Verbindung in Gegenwart eines basischen Kondensationsmittels, dadurch gekennzeichnet, daß als basisches Kondensationsmittel 1,5-Diazabicyclo[4,3,0]non-5-en (DBN) oder 1,8-Diazabicyclo[5,4,0]undec-7-en (DBU) verwendet wird.

2. Verfahren nach Anspruch 1, dadurch gekennzeichnet, daß die Umsetzung in einem aprotischen, dipolaren Lösungsmittel durchgeführt wird.

**Claims**

1. Process for the production of a α-pivaloyl acetanilide or a α-benzoyl acetanilide substituted in the α-position, by reacting the corresponding α-halogen compound of the formula

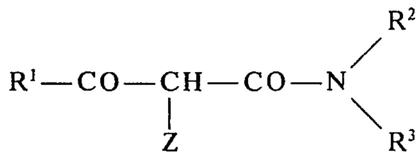

wherein

Z denotes halogen,
$R^1$ denotes tert. butyl or aryl,
$R^2$ denotes hydrogen or $C_1-C_5$-alkyl and
$R^3$ denotes aryl,

with a phenol or an NH-acidic organic compound in the presence of a basic condensation agent, characterised in that 1,5-diazabicyclo-[4,3,0]-non-5-ene (DBN) or 1,8-diazabicyclo-[5,4,0]-undec-7-ene (DBU) is used as the basic condensation agent.

2. Process according to Claim 1, characterised in that the reaction is carried in an aprotic, dipolar solvent.

**Revendications**

1. Procédé pour la préparation d'un α-pivaloylacétanilide ou α-benzoylacétanilide substitué en position α par réaction du composé α-halogéné correspondant de formule

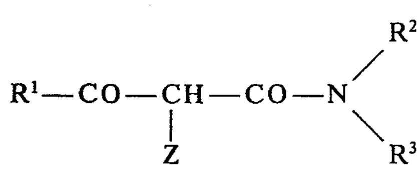

dans laquelle

Z représente un halogène,
$R^1$ représente un groupe tert.butyle ou aryle,
$R^2$ représente l'hydrogène ou un groupe alkyle en $C_1-C_5$,
$R^3$ représente un groupe aryle,

avec un phénol ou un composé organique à NH acide, en présence d'un agent de condensation basique, caractérisé en ce que l'on utilise comme agent de condensation basique le 1,5-diazabicyclo[4,3,0]non-5-ène (DBN) ou le 1,8-diazabicyclo[5,4,0]undéc-7-ène (DBU).

2. Procédé selon la revendication 1, caractérisé en ce que l'on effectue la réaction dans un solvant dipolaire aprotique.